# EUROPEAN PATENT APPLICATION

(11) **EP 2 269 546 A1**
(43) Date of publication of application: **05.01.2011**
(21) Application number: 09172908.7
(22) Date of filing: 13.10.2009
(51) Int. Cl.: A61F 7/02

(54) **Occlusion resistant and/or multilayer treatment pad**

(30) Priority: 03.07.2009 EP 09164533
(71) Applicant: Dewaegenaere, Levi Emmerik A., 2970 s'-Gravenwezel (BE)
(72) Inventor: Dewaegenaere, Levi Emmerik A., 2970 s'-Gravenwezel (BE)
(74) Representative: Vossius & Partner

(57) **Abstract**

The invention provides for a therapeutic pad for thermal treatment of a human or animal body part. The pad comprises two outer layers forming a fluid communication channel therebetween, a fluid inlet port and a fluid outlet port adapted to connect the fluid communication channel with an external fluid reservoir, and a layer structure between the two outer layers (Fig. 4).

## Description

The invention relates to a thermal treatment pad having a fluid communication channel to thermally treat a human or animal body part. The pad further comprises an inner layer structure enhancing stability of its shape and avoiding occlusion of the channel, when the pad is in use.

### FIELD OF THE INVENTION

The present invention is in the technical field of therapeutic treatment of the human or animal body

More particularly, the present invention is in the technical field of therapeutic treatment of the human or animal body by means of a flexible pad applied to the body part to be treated. The pad may be attached to a medical device, which ensures the circulation through the pad of a fluid at controlled and actively regulated temperature, pressure and flow.

### BACKGROUND OF THE INVENTION

Particular ailments or diseases require treatment by the application of cold or heat to a body part, which has a particular beneficial medical outcome, according to the treatment protocol applied (e.g. intensity and duration of treatment). In the past this was accomplished, for example, by applying ice or cold liquid to the affected area for cryotherapy, and applying a heat source, such as a heated towel, for heat therapy. It has been discovered that using a thermal pad considerably increases the efficiency and convenience of such treatment. The treatment of the body part with a thermal pad is carried out through controlled and actively regulated thermal energy exchange between the pad and the body part to which it is applied. The treatment efficiency of a pad maybe optimal when the heat exchange surface has maximal contact with the body location being treated to facilitate heat exchange between the body part and the pad. However, the performance of prior-art pads is unsatisfactory. In particular, it is necessary to maintain a high pressure of fluid within the pad in order to maximize the contact between the pad and skin (i,e. the effective treatment area). However, the increased pressure within the pad may cause it to burst. An alterative may be to create a pad with stronger and more rigid material, however, this may result in reduced flexibility and a consequent reduction of the effective treatment area.

In particular, in the application domain of interest, the active control of temperature, flow and pressure may become important for the positive outcome of the treatment. In some cases, higher fluid pressures (or higher flows, that can result in higher pressures inside the therapeutic pad ) are necessary. At the same time, maximizing the surface area in contact with the human body is also necessary. The end result may be a failure of the material of the material/outer layer of the pad. One further solution may be to implement a thicker material/outer layer. However, in doing so, the flexibility characteristics of the therapeutic pad may be impacted so that the therapeutic pad will become less flexible, and/or the surface area in contact with the human body has adversely to be reduced.

A further aspect may be that the performance of prior-art pads is unsatisfactory with respect to maintaining its outer shape: in particular, the flow of fluid within the pad may become restricted by when it conforms or bends to the particular body part to be treated. Further, when the pad is drained of fluid from a fluid outlet by applying suction on the fluid outlets in the pad, the negative pressure can cause the internal layers of the pad to come into contact with each other in an uneven fashion, which results in fluid being trapped in 'pockets' within the pad.

US Patent No. 5,662,695 by Mason, discloses a method of providing a rigid material around the fluid outlet in order to resist occlusion of the fluid. However, this method does not prevent fluid pockets being formed by negative pressure, and does not prevent occlusion throughout the entire pad. US Patent Application No. 11/803,704 by Mason attempts to overcome this problem by providing a tube which extends a short distance into the pad from the fluid outlet. Again, this method does not overcome the problem of pockets caused by negative pressure when draining the patch, and does not prevent occlusion throughout the pad entire pad, which may be caused by bending.

Accordingly, there is a need for a pad to overcome these problems in the prior art.

### OBJECTS OF THE INVENTION

The object of the present invention is to provide a therapeutic pad that allows the flow of fluid within such pad while maintaining flexibility and resistance to pressure.

It is a further object of this invention to provide a therapeutic pad that maximizes the surface area which is in contact with a patient while allowing increased pressure in the flow of fluid at a non-ambient temperature within such pad.

The object of the present invention is to provide a therapeutic pad that allows the drainage of fluid in a manner that prevents blockages caused by negative pressure within such pad.

It is a further object of this invention to provide a therapeutic pad that minimizes the obstruction of fluid flow within such pad when conforming to a flexible surface.

It is a further object of this invention to provide a therapeutic pad that allows unrestricted flow of fluid within such pad in order to maximize the therapeutic treatment of the affected area by such pad.

Further objects and advantages of the present invention will be disclosed and become apparent from the following description.

### SUMMARY OF THE INVENTION

These objects are achieved with the features of the claims.

In one embodiment, the present invention is a pad for the therapeutic treatment of the human body that allows the use of high-pressure circulation of the fluid while maximizing the active surface area, i.e. the area constantly in contact with the human body part under treatment. For example, the present invention uses occlusion means, as described further in detail below, to assist free circulation of fluid within said pad and further enables the draining of such pad by a medical device connected which circulates such fluid within said pad.

According to the present invention, a therapeutic pad for thermal treatment of a human or animal body part is provided. The pad comprises two outer layers forming a fluid communication channel therebetween, a fluid inlet port and a fluid outlet port adapted to connect the fluid communication channel with an external fluid reservoir, and a layer structure between the two outer layers.

An external fluid reservoir maybe any kind of device or container, adapted to provide fluid to the fluid communication channel of the pad and not being integrally formed by the pad. In particular, the reservoir a hydraulic device and/or a pump for fluid. For example, the hydraulic device or pump may be provided with tubes which end(s) fit into the inlet and/or outlet ports of the pad, for example, by means of a plug/socket connection. In doing so, the hydraulic device or pump provides the pad with fluid to actuate the thermal treatment. In a particular embodiment, the layer structure is located in and/or surrounds the fluid communication channel.

In a particular embodiment, the layer structure is made from a flexible plastic material, PU, PVC, a foam, a fibrous material, a nylon mesh, and/or a nonwoven fabric or web.

A nonwoven fabric is a fabric like material made from long fibers, bonded together by chemical, mechanical, heat or solvent treatment. The tenn is used in the textile manufacturing industry to denote fabrics, such as felt, which are neither woven nor knitted.

Nonwoven fabrics maybe manufactured by putting small fibers or filaments together in the form of a sheet or web (similar to paper on a paper machine), and then binding them either mechanically, with an adhesive, or thermally (by applying binder in the form of powder, paste, or polymer melt) and melting the binder onto the web by increasing temperature).

In a particular embodiment, the layer structure comprises two layers.

In a particular embodiment, the two layers of the layer structure are made of the same material.

In a particular embodiment, the two layers of the layer structure surround the fluid communication system.

In a particular embodiment, the layer structure is a single layer.

In a particular embodiment, the pad comprises two inner layers encapsulating the single layer of the layer structure and are provided between the two outer layers.

In a particular embodiment, the two inner layers are made from the same material, and the single layer of the layer structure is made from a different material than the two inner layers.

In a particular embodiment, the layer structure is made from a nonwoven fabric or web.

In a particular embodiment, the outer layers are films.

Films are extruded layers made from a plastic, particularly synthetic, material.

In a particular embodiment, the outer layers are made from PU and/or PVC.

In a particular embodiment, the thickness of the outer layers is between 10 µm and 5mm, particularly between 100 µm and 3 mm, more particularly between 1 mm and 2 mm.

In a particular embodiment, the outer layers are welded to form the fluid communication channel.

In a particular embodiment, the layer structure is co-welded together with the outer layers.

A co-welding may be advantageous, since only one manufacturing step is needed to form the therapeutic pad from the outer layers and the layer structure. In this case, the welding points and/or lines of the outer layers and the layer structure are co-located.

The pad according to the invention provides for a quite homogeneous circulation of the fluid within the pad to provide for a homogeneous temperature distribution throughout the pad and the pad surface contacting the patient's skin.

The use of additional outer layers reinforces the pad and allows an increased pressure.

### BRIEF DESCRIPTION OF THE DRAWINGS

Fig. 1 schematically shows a top view of a therapeutic pad;
Fig. 2 schematically shows a side section view of a therapeutic pad;
Fig. 3 schematically shows a particular side section view of a therapeutic pad;
Fig. 4 schematically shows a particular side section view of a therapeutic pad;
Fig. 5 schematically shows a therapeutic pad section in top view; and
Fig. 6 schematically shows a perspective side section view of a therapeutic pad.

### DETAILED DESCRIPTION OF THE INVENTION

Various embodiments of the present invention are described hereinafter with reference to the figures. It should be noted that the figures are not drawn to scale and elements of similar structures or functions are represented by like reference numerals throughout the figures. It should also be noted that the figures are only intended to facilitate the description of specific embodiments of the invention. In addition, an aspect described in conjunction with a particular embodiment of the present invention is not necessarily limited to that embodiment and can be practiced in any other embodiments of the present invention.

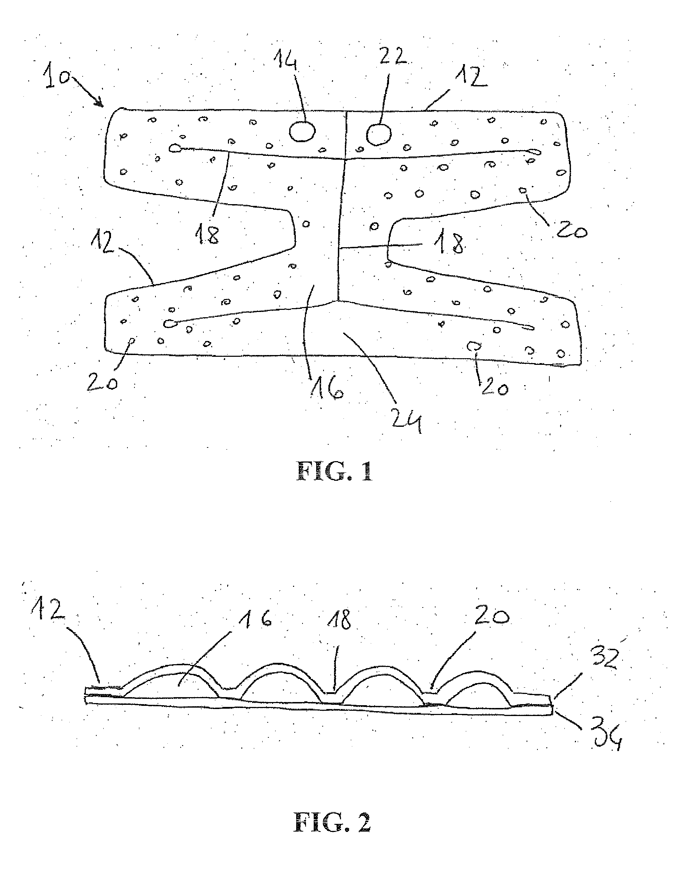

Referring now to the invention in more detail, Fig. 1 shows a top view of a therapeutic pad (10). The shape of the therapeutic pad (10) is not relevant in the context of this embodiment and may depend on the application and specific body part to be treated. The depicted therapeutic pad (10) in Fig. 1 may be used for knee application. Other shapes are possible and may be preferred for other body parts, for example, but not limited to shoulder and face.

The therapeutic pad (10) is outlined by externally enclosing welding lines (12). The therapeutic pad receives the fluid from a medical device from one fluid inlet (14). The fluid circulates in the therapeutic pad through internal communicating channels (16), delimited by internal welding lines (18) and welding points (20). Finally the fluid returns to the medical device through one fluid outlet (22).

Fig. 2 shows a side section of a therapeutic pad (10). In Fig. 2 there are shown 2 films (32, 34) welded together according to the outline and internal communicating channels (16), delimited by internal welding lines (18) and welding points (20) as shown in Fig. 2.

The construction details of the invention as shown in Fig. 1 and Fig. 2 are that the therapeutic pad (10) is made of two films (32, 34) of material like PU (palyurethane),PVC (polyvinyl chloride). The thickness of the material is within the range of 10 micro to 5 millimeters. The films are welded together according to the outline and internal communicating channels (16) as shown in Fig. 1. The welding is performed through techniques such as ultra high frequency welding.

Again referring to Fig. 1, an area (24) of the surface of the therapeutic pad (10) is provided which is missing welding points or lines. For example, this is done to achieve a bigger service area (i.e. area in contact with the human body). In view of some particular applications, the area (24) may be less resistant (with respect to the actually needed treatment of the patient) to higher fluid pressures inside the therapeutic pad (10), because a higher pressure (caused by the bigger surface) is not properly restrained since there are less welding points or lines.

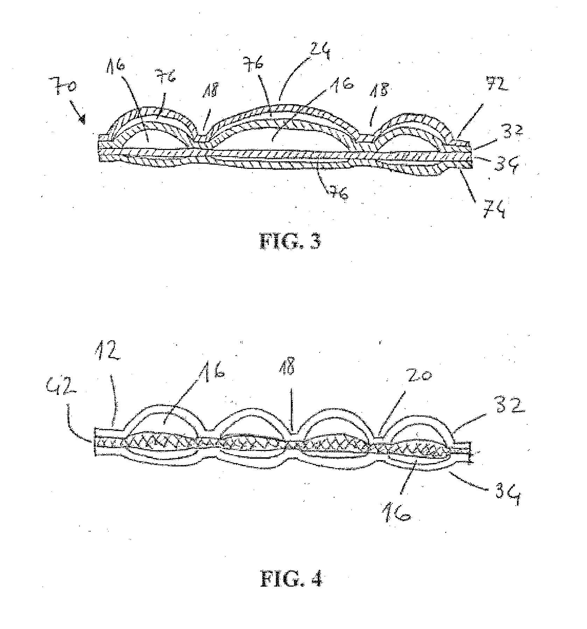

Fig. 3 shows the side section of a further therapeutic pad (70); the therapeutic pad (70) is constructed by using four layers of a film of PU, PVC or similar material (32, 34, and 72, 74), of the same thickness as the films (32, 34) of the therapeutic pad (10) described in Fig. 1 and Fig. 2. The outer layers (72, 74) are welded to the inner layers (32, 34 respectively) using technologies such as ultra high frequency welding. Between the outer layers (72, 74) and the inner layers (32, 34), in the regions (76) were there are no welding lines or welding points, air, or any other fluid (liquid and/or gas) or a nonwoven layer may be present.

The advantages of the embodiment depicted in Fig. 3, include, without limitation, the ability to sustain higher fluid pressure levels of the fluid inside the therapeutic pad (70,16), with higher surface areas (24) in contact with the human body, keeping the same flexibility characteristics of the therapeutic pad (10) described in Fig. 1 and Fig. 2.

In more general terms, a preferred embodiment of the present invention is a therapeutic pad with two outer layers and two inner layers of PU, PVC or similar material, with the fluid circulating between the inner layers.

Fig. 4 shows an occlusion body (42) inserted between two layers (32, 34) of a therapeutic pad, for example, or the pad as shown in Figs. 1 and 2. In doing so, the occlusion body (42) is in contact with the circulating fluid within the therapeutic pad (10). In Fig. 4 the therapeutic pad (10) is made of only two films (32, 34), except for the occlusion body (42). In Fig. 4 the occlusion body (42) is an additional layer of foam or filter material, or mesh fabric or nonwoven fabric or web. The occlusion body layer (42) of foam or fiber material may be welded together with the other layers (32, 34). In a particular embodiment, the occlusion body (42) may contain at least two layers, wherein at least one layer is a nonwoven layer. For example, the nonwoven layer may be the layer contacting the two layers (32, 34), wherein the other layer of the occlusion body (42) merely contacts the nonwoven layer and - in use - the fluid circulating through the pad.

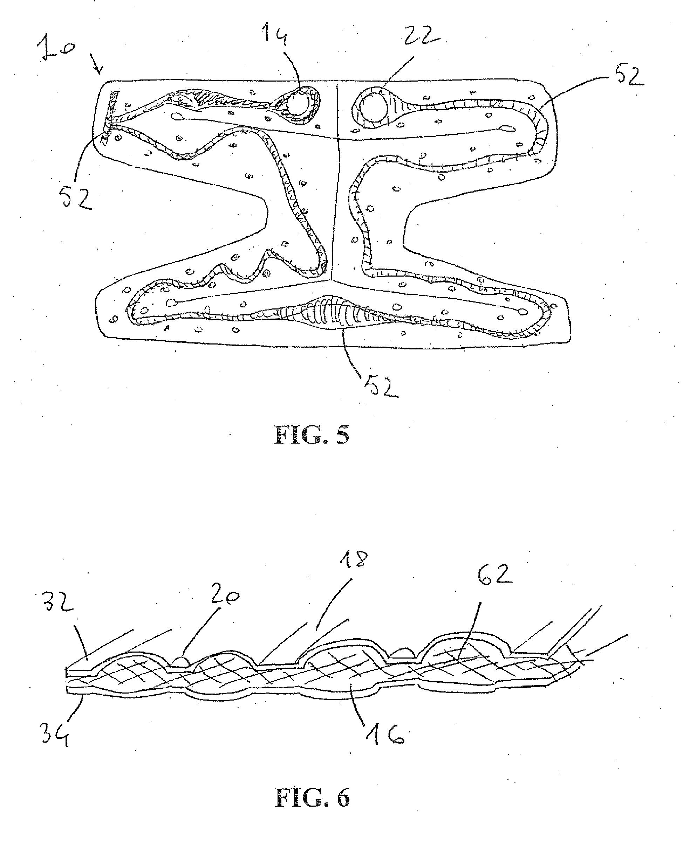

Fig. 5 shows an occlusion body (52) inserted between the other layers as discussed with reference to Fig. 4, so to be in contact with the circulating fluid within the therapeutic pad (10). In Fig. 5 the occlusion body (52) is an additional wire or mesh of a material such as flexible plastic, foam or fiber material. The wire or mesh (52) is placed inside the therapeutic pad (10) before the welding, and is enclosed inside the therapeutic pad (10) after the welding has been performed. The occlusion body (52) is not welded to the therapeutic pad (10), but only included in it. In this case, the occlusion body (52) is encapsulated by the layers (32, 34) of the pad and does not have any common welding points or lines in common with the pad layers (32, 34).

Fig. 6 shows an additional layer of material like a nylon mesh (62), which can be welded in contact or alternatively not in contact with the circulating fluid. When the additional layer of nylon mesh is not in contact with the circulating fluid, it means that the therapeutic pad (10) is constructed with more than two films af pU, PVC or similar material.

The advantages of the inventions depicted in Fig. 4, Fig. 5 and Fig. 6 include, without limitation, the ability to drain the fluid inside the therapeutic pad. In previous therapeutic pads, the negative pressure created by the draining operation performed by the medical device would have the layers of the therapeutic pad stick together, therefore not allowing all the fluid, or a part of it, to be drained in the medical device.

The advantages of the inventions depicted in Fig. 4, Fig. 5 and Fig. 6 include, without limitation, the avoidance of occlusions and obstacles to the circulation of fluid within the therapeutic pad by, for example, kinking the therapeutic patch.

In more general terms, the above described embodiment of the present invention is a therapeutic pad with any kind of occlusion body inside it.

While the invention has been illustrated and described in detail in the foregoing description, such illustration and description are to be considered illustrative or exemplary and non-restrictive; the invention is thus not limited to the disclosed embodiments. Features mentioned in connection with one embodiment described herein may also be advantageous as features of another embodiment described herein without explicitly showing these features. Variations to the disclosed embodiments can be understood and effected by those skilled in the art and practicing the claimed invention, from a study of the disclosure and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures can not be used to advantage.

## Claims

1. Therapeutic pad (10; 70) for thermal treatment of a human or animal body part, comprising:
two layers (32,34) forming a fluid communication channel therebetween;
a fluid inlet port (14) and a fluid outlet port (22) adapted to connect the fluid communication channel with an external fluid reservoir; and
an occluding means for substantially preventing contact between the two layers (32, 34).

2. Pad of claim 1, comprising a layer structure (42; 52; 62; 72, 74) between the two outer layers.

3. Pad of claim 2, wherein the layer structure is located in and/or surrounds the fluid communication channel.

4. Pad of claim 2 or 3, wherein the layer structure is made from a flexible plastic material, PU, PVC, a foam, a fibrous material a nylon mesh, and/or a nonwoven fabric or web.

5. Pad of claim 2, 3 or 4, wherein the layer structure is a single layer (42).

6. Pad of any one of claims 1 to 5, comprising two outer layers (72, 74) made of the same material.

7. Pad of claim 6, wherein the two outer layers (72, 74) surround the fluid communication system provided by the two layers (32, 34).

8. Pad of claim 7, wherein two inner layers (32, 34) encapsulate the single layer (42) of the layer structure and are provided between the two outer layers (72, 74).

9. Pad of claim 8, wherein the two inner layers are made from the same material, and wherein the single layer of the layer structure is made from a different material than the two inner layers.

10. Pad of any one of claims 2 to 9, wherein the layer structure is made of a nonwoven fabric or web.

11. Pad of any one of the preceding claims, wherein the outer layers are films.

12. Pad of any one of the preceding claims, wherein the outer layers are made from PU and/or PVC.

13. Pad of any one of the preceding claims, wherein the thickness of the outer layers is between 10 µm and 5mm.

14. Pad of any one of the preceding claims, wherein the outer layers are welded to form the fluid communication channel.

15. Pad of claim 14, wherein the layer structure is co-welded together with the outer layers.
